# EUROPEAN PATENT APPLICATION

(11) **EP 3 763 727 A1**
(43) Date of publication of application: **13.01.2021**
(21) Application number: 19754897.7
(22) Date of filing: 18.02.2019
(51) Int. Cl.: C07K 7/08, C07K 16/18, A61K 45/00, A61P 35/00, A61K 38/00, A61K 39/00

(54) **VACCINE COMPRISING EPITOPE OF HEAT SHOCK PROTEIN, AND USE THEREOF**

(30) Priority: 19.02.2018 KR 20180019123; 15.02.2019 KR 20190018119
(71) Applicant: Korea University Research and Business Foundation, Seoul 02841 (KR)
(72) Inventor: PARK, Kyong Hwa, Seoul 02702 (KR); KANG, Jin Ho, Seoul 02850 (KR)
(74) Representative: ZBM Patents - Zea, Barlocci & Markvardsen
(86) International application number: PCT/KR2019/001898
(87) International publication number: WO 2019/160383

(57) **Abstract**

The present invention relates to a vaccine comprising an epitope of a heat shock protein 90 and a combination therapy thereof. According to the present invention, if a vaccine composition containing a polypeptide that is an epitope of heat shock protein 90 represented by the amino acid sequence of SEQ ID NO: 1 or 2 is used, it can be used universally in most patients, industrialization is easy, and anti-tumor effect Is remarkably excellent, thereby being economically used to prevent and treat cancer.

## Description

### Field of the Invention

The present invention relates to a vaccine comprising an epitope of a heat shock protein 90 and a combination therapy thereof. In more detail, it relates to a polypeptide that is an epitope of heat shock protein 90 represented by the amino acid sequence of SEQ ID NO: 1 or 2, a vaccine composition comprising the same, a method for treating or preventing cancer using the composition.

### Background of the Invention

In modern times, many diseases have become readily treatable, and diseases that cannot be cured have almost disappeared. However, unlike other disease treatments, cancer is very difficult and requires complex treatment, and the complex treatments are also not completely effective. Recently, immunotherapy methods are emerging. Immunotherapy is a method of treating cancer by using the immune response in the patient's body. Cancer can be prevented through this immunotherapy method. According to the principle of a vaccine, cancer immunotherapy is a method of activating cancer-specific immune cells by administering an antigen that causes cancer, and then causing the activated immune cells to specifically attack cancer in the body. In addition, when a cancer-specific antigen is administered into the body without cancer, the immune cells that have not been activated are activated as cancer-specific memory immune cells, thereby specifically attacking the cancer cells in case of cancer.

Heat shock protein 90 (Hsp90) is an ATPase-dependent molecular chaperone that is required for protein folding, maturation, and stabilization of conformational forms of many "client" proteins (Young et al., 2000; Kamal et al. al., 2003). Hsp90 interacts with several proteins involved in CRPC, including growth factor receptors, cell cycle regulators, and Akt pathway, androgen receptor (AR) or Raf-1 (Whitesell et al., 2005; Takayama et al., 2003). Tumor cells express higher Hsp90 levels compared to benign cells (Kamal et al., 2003; Chiosis et al., 2003), and Hsp90 inhibition occurs as an important target in CRPC and other cancers. Many Hsp90 inhibitors have developed while targeting their ATP-binding pockets, including natural compounds such as geldanamycin and its analogs, or synthetic compounds. These agents have been shown to inhibit Hsp90 function, leading to apoptosis in preclinical studies of colon, breast, PCa and other cancers (Kamal et al., 2003; Solit et al., 2003; Solit et al., 2002).

Recently, HSPPC-96 (Oncophage®, Antigenics, Inc., New York, NY, USA) containing autologous tumor cell-derived gp96 HSP peptide complex has excellent therapeutic effect when combined with IL-2 in stage 4 metastatic kidney cancer patients. There is no effect of prolonging survival in phase 3 clinical trials compared to conventional treatments in stage 4 melanoma patients, but safety, effective induction of immune responses, and long-term immune memory have been reported. However, since it is derived from autologous tumor cells, it is not applicable to patients who are difficult to obtain tumor cells. It is difficult to commercialize because the vaccine production cost is high and standardization of the manufacturing process and effect is difficult, and the epitope of the tumor-specific protein is not known. There is a difficult problem that monitoring is impossible.

### BRIEF SUMMARY OF THE INVENTION

Accordingly, the present inventors have made a thorough effort to find a vaccine composition that can be used universally in the majority of patients and has an excellent anti-tumor effect.

The present invention has been validated, by confirming that when using a vaccine composition containing a multi-peptide that is an epitope of heat shock protein 90 represented by the amino acid sequence of SEQ ID NO: 1 or 2, it can be widely used in most patients, industrialization is easy, and antitumor effect is excellent.

An object of the present invention is to provide a polypeptide that is an epitope of heat shock protein 90.

Another object of the present invention is to provide a vaccine composition for the treatment or prevention of cancer comprising the polypeptide as an active ingredient.

Another object of the present invention is to provide an anticancer composition comprising the polypeptide as an active ingredient.

Another object of the present invention is to provide a method for treating or preventing cancer comprising administering the vaccine composition to a cancer patient.

Another object of the present invention is to provide an antibody that specifically recognizes the polypeptide.

In order to achieve the above object, the present invention provides a polypeptide that is an epitope of heat shock protein 90 represented by the amino acid sequence of SEQ ID NO: 1 or 2.

The present invention also provides a gene encoding the polypeptide, a recombinant vector containing the gene, and a recombinant microorganism into which the gene or the recombinant vector has been introduced.

The present invention also provides a method for preparing the polypeptide comprising the steps of: (a) culturing the recombinant microorganism to produce the polypeptide; and (b) obtaining the produced polypeptide.

The present invention also provides a vaccine composition for treating or preventing cancer comprising the polypeptide as an active ingredient, and a method for treating or preventing cancer comprising administering the vaccine composition to a cancer patient.

The present invention also provides an antibody that specifically recognizes the epitope of heat shock protein 90 represented by the amino acid sequence of SEQ ID NO: 1 or 2.

The vaccine composition of the present invention containing the epitope of the heat shock protein 90 is universally usable in the majority of patients, is easy to industrialize, and has remarkably excellent antitumor effects, so it can be economically used to prevent and treat cancer.

### BRIEF DESCRIPTION OF THE DRAWINGS/FIGURES

Fig. 1 is a result of predicting epitopes of Hsp 90 protein using a computer algorithm program in the present invention.
Fig. 2 is a result of confirming the immunogenicity of the Hsp 90 multi-peptide vaccine of the present invention.
Fig. 3 is a result of confirming the anti-tumor effect of the Hsp 90 multi-peptide vaccine of the present invention in the mouse model.
Fig. 4 is a result of confirming the antitumor effect of the combined treatment of HSP90 multi-peptide vaccine, STING agonist, and CTLA-4 inhibitor in a mouse model.

### DETAILED DESCRIPTION OF THE INVENTION

In the present invention, it was confirmed that the vaccine composition containing the epitope polypeptide of heat shock protein 90 (Hsp90) represented by the amino acid sequence of SEQ ID NO: 1 or 2 induces the Th1 immune response and has excellent anti-tumor effects.

Therefore, in one aspect, the present invention relates to a polypeptide that is an epitope of heat shock protein 90 represented by the amino acid sequence of SEQ ID NO: 1 or 2.

In the present invention, the term "epitope" refers to a set of amino acid residues at the antigen binding site recognized by a specific antibody, or by a T cell receptor protein and/or a major histocompatibility complex (MHC) receptor in T cells. An epitope is a molecule that forms a site recognized by an antibody, a T cell receptor, or an HLA molecule, and refers to a primary, secondary and tertiary peptide structure, or charge.

In an embodiment of the present invention, 12 15-mer peptide sequences that are expected to have good binding affinity to the most common MHC class II allele using 5 algorithm programs for Hsp 90 full sequences were selected. Thereafter, two types of epitopes having good binding affinity to the MHC class II allele and excellent antitumor effect were obtained.

In another aspect, the present invention relates to a gene encoding the polypeptide.

In the present invention, the gene may be characterized in that it is represented by the nucleotide sequence of SEQ ID NO: 3 or SEQ ID NO: 4.

In another aspect, the present invention relates to a recombinant vector containing the gene and a recombinant microorganism into which the gene or the recombinant vector has been introduced.

In another aspect, the present invention relates to a method for producing the polypeptide comprising the steps of (a) culturing the recombinant microorganism to produce the polypeptide; and (b) obtaining the produced polypeptide.

In the present invention, the vector refers to a DNA sequence linked to a suitable control sequence so that the target protein can be expressed in a suitable host cell. The control sequence may include a promoter capable of initiating transcription, any operator sequence for regulating such transcription, a sequence encoding a suitable mRNA ribosome binding site, and a sequence controlling the termination of transcription and translation. It can be manufactured in various ways, depending on the purpose. The promoter of the vector can be constitutive or inducible. Vectors can be transformed into a suitable host and then replicate or function independently of the host genome, or can be integrated into the genome itself.

The vector used in the present invention is not particularly limited as long as it can replicate among host cells, and any vector known in the art may be used. Examples of commonly used vectors include natural or recombinant plasmids, phagemids, cosmids, viruses and bacteriophages. For example, pWE15, M13, λMBL3, λMBL4, λIXII, λASHII, λAPII, λt10, λt11, Charon4A, and Charon21A can be used as a phage vector or a cosmid vector, and as a plasmid vector, pBR system, pUC system, pBluescript II system, pGEM system, pTZ system, pCL system and pET system can be used. The vector usable in the present invention is not particularly limited, and a known expression vector may be used.

The term "expression control sequence" means a DNA sequence essential for the expression of a coding sequence operably linked in a particular host organism. Such regulatory sequences include promoters for effecting transcription, any operator sequences for regulating such transcription, sequences encoding suitable mRNA ribosome binding sites, and sequences that regulate termination of transcription and translation. For example, regulatory sequences suitable for prokaryotes include a promoter, optionally an operator sequence, and a ribosome binding site. Regulatory sequences suitable for eukaryotic cells include promoters, polyadenylation signals and enhancers. The factor that most affects the amount of gene expression in the plasmid is the promoter. As a promoter for high expression, an SRa promoter, a cytomegalovirus-derived promoter, and the like are preferably used.

In order to express the DNA sequence of the present invention, any of a wide variety of expression control sequences can be used in the vector. Examples of useful expression control sequences include early and late promoters of SV40 or adenovirus, lac system, trp system, TAC or TRC system, T3 and T7 promoters, major operator and promoter regions of phage lambda, regulatory regions of FD protein, promoters for 3-phosphoglycerate kinase or other glycolytic enzymes, promoters of the phosphatase, for example Pho5, promoters of the yeast alpha-crossing system, and other sequences of constructs and inductions known to regulate the expression of genes of prokaryotic or eukaryotic cells or their viruses, and various combinations thereof. The T7 RNA polymerase promoter φ10 can be usefully used to express protein NSP in E. coli.

Nucleic acids are "operably linked" when placed in a functional relationship with another nucleic acid sequence. It may be a gene and regulatory sequence(s) linked in a manner that allows gene expression when an appropriate molecule (eg, a transcriptional activating protein) is bound to the regulatory sequence(s). For example, DNA for a pre-sequence or secretory leader is operably linked to the DNA for a polypeptide when expressed as a whole protein that participates in the secretion of the polypeptide; the promoter or enhancer is operably linked to the coding sequence if it affects the transcription of the sequence; the ribosome binding site is operably linked to the coding sequence if it affects the transcription of the sequence; or the ribosome binding site is operably linked to a coding sequence when arranged to facilitate translation. In general, "operably linked" means that the linked DNA sequence is in contact, and, in the case of a secretory leader, is brought into contact and within the reading frame. However, the enhancer does not need to be in contact. The ligation of these sequences is carried out by ligation (linkage) at convenient restriction enzyme sites. If such a site does not exist, a synthetic oligonucleotide adapter or linker according to a conventional method is used.

The term "expression vector" as used herein is a recombinant carrier into which a fragment of a heterologous DNA is inserted, and generally refers to a fragment of doublestranded DNA. Here, heterologous DNA refers to DNA that is not naturally found in host cells. Once in the host cell, the expression vector can replicate independently of the host chromosomal DNA and several copies of the vector and its inserted (heterologous) DNA can be generated.

As is well known in the art, in order to increase the expression level of a transfected gene in a host cell, the gene must be operably linked to transcriptional and translational expression control sequences that exert a function in the selected expression host. Preferably, the expression control sequence and the corresponding gene are included in a single expression vector containing a bacterial selection marker and a replication origin. When the expression host is a eukaryotic cell, the expression vector should further contain an expression marker useful in the eukaryotic expression host.

In order to express the gene encoding the polypeptide of the present invention, a wide variety of expression host/vector combinations can be used. Expression vectors suitable for eukaryotic hosts contain expression control sequences derived from, for example, SV40, bovine papillomavirus, anenovirus, adeno-associated virus, cytomegalovirus and retrovirus. Expression vectors that can be used for bacterial hosts include bacterial plasmids obtained from E. coli such as pBluescript, pGEX2T, pUC vector, colE1, pCR1, pBR322, pMB9 and derivatives thereof; plasmids having a wider host range such as RP4; phage DNA, which can be exemplified by a wide variety of phage lambda derivatives such as φgt10 and λgt11, NM989; and other DNA phages such as M13 and filamentous single-stranded DNA phage. Expression vectors useful for yeast cells are 2µ plasmids and derivatives thereof. A vector useful for insect cells is pVL 941.

Host cells transformed or transfected with the above-described expression vector constitute another aspect of the present invention. As used herein, the term "transformation" means that DNA is introduced into a host so that the DNA becomes replicable as an extrachromosomal factor or by completion of chromosomal integration. As used herein, the term "transfection" means that the expression vector is accepted by the host cell, whether or not any coding sequence is actually expressed.

The host cell of the present invention is a recombinant microorganism into which a vector having a polynucleotide encoding one or more target proteins is introduced; or a polynucleotide encoding one or more target proteins is introduced, and the polynucleotide is integrated into a chromosome to express the target protein. It may be a prokaryotic or eukaryotic cell. In addition, a host having a high DNA introduction efficiency and a high expression efficiency of the introduced DNA is usually used. Known eukaryotic and prokaryotic hosts such as E. coli, Pseudomonas, Bacillus, Streptomyces, fungi, yeast, insect cells such as Spodoptera frugiperda (SF9), animal cells such as CHO and mouse cells, African green monkey cells such as COS 1, COS 7, BSC 1, BSC 40 and BMT 10, and tissue cultured human cells are examples of host cells that can be used. In the case of using COS cells, since SV40 large T antigen is expressed in COS cells, the plasmid with the replication initiation point of SV40 is to exist as an episome of multiple copies in the cell. Higher than usual expression can be expected. The introduced DNA sequence may be obtained from the same species as the host cell, may be of a different species than the host cell, or it may be a hybrid DNA sequence comprising any heterologous or homologous DNA.

Of course, it should be understood that not all vectors and expression control sequences function equally in expressing the DNA sequence of the present invention. Likewise, not all hosts function equally for the same expression system. However, those skilled in the art can make an appropriate selection among various vectors, expression control sequences, and hosts without departing from the scope of the present invention without undue experimental burden. For example, when choosing a vector, you must consider the host, because the vector must be replicated within it. The number of copies of the vector, the ability to regulate the number of copies, and the expression of other proteins encoded by the vector, such as antibiotic markers, should also be considered. In selecting the expression control sequence, several factors must be considered. For example, the relative strength of the sequence, controllability, and compatibility with the DNA sequence of the present invention, etc., should be considered in particular with regard to possible secondary structures. Single-celled hosts should be selected, considering factors such as the selected vector, toxicity of the product encoded by the DNA sequence of the present invention, the secretion characteristics, the ability to accurately fold the protein, culture and fermentation requirements, the easiness in the purification of the product encoded by the present DNA sequence from the host. Within the range of these variables, one of ordinary skill in the art can select various vector/expression control sequence/host combinations capable of expressing the DNA sequence of the present invention in fermentation or large-scale animal culture. As a screening method for cloning the cDNA of the polypeptide of the present invention by expression cloning, a binding method, a panning method, a film emulsion method, or the like can be applied.

In the present invention, as a method of inserting the gene onto the chromosome of a host cell, a commonly known gene manipulation method can be used. The example of the non-viral delivery method includes a cell puncture method, lipofection, microinjection, ballistic method, virosome, liposome, Immunoliposomes, polyvalent cations or lipids: nucleic acid conjugates, naked DNA, artificial virons and chemical-promoting DNA influx. Sonoporation, for example, a method using the Sonitron 2000 system (Rich-Mar) can also be used for the delivery of nucleic acids, and other representative nucleic acid delivery systems include the method such as Amaxa Biosystems (Cologne, Germany), Maxcyte, ATx (Rockville, Maryland) and BTX Molesular Syetem (Holliston, MA). The lipofection method is specified in U.S. Patent No. 5,049,386, U.S. Patent No. 4,946,787 and U.S. Patent No. 4,897,355, and lipofection reagents are commercially available from for example, TransfectamTM and LipofectinTM. Cationic or neutral lipids suitable for effective receptor-recognition lipofection of polynucleotides include Felgner's lipids (WO91/17424 and WO91/16024) and can be delivered to cells through ex vivo introduction and into target tissues through in vivo introduction. Methods for preparing lipid:nucleic acid complexes including targeting liposomes such as immunolipid complexes are well known in the art (Crystal, Science., 270:404-410, 1995; Blaese et al., Cancer Gene Ther., 2:291 -297, 1995; Behr et al., Bioconjugate Chem., 5:382389, 1994; Remy et al., Bioconjugate Chem., 5:647-654, 1994; Gao et al., Gene Therapy., 2:710- 722, 1995; Ahmad et al., Cancer Res., 52:4817-4820, 1992; U.S. Patent 4,186,183; U.S. Patent 4,217,344; U.S. Patent 4,235,871; U.S. Patent 4,261,975; U.S. Patent 4,485,054 ; U.S. Patent No. 4,501,728; U.S. Patent No. 4,774,085; U.S. Patent No. 4,837,028; U.S. Patent No. 4,946,787).

The affinity of the retrovirus can be changed by integration with the outer envelope protein, thus expanding the type of target cell. A lentiviral vector is a retroviral vector that transduces or infects non-dividing cells to produce high viral titers. The target tissue determines the retroviral gene delivery system. Retroviral vectors contain cis-acting long terminal repeats capable of packaging 6-10 kb external sequences. The minimal cis-acting LTR, sufficient for cloning and packaging of the vector, can be used to integrate therapeutic genes into target cells for permanent transgene expression. Widely used retroviral vectors include murine leukemia virus (MuLV), gibbon leukemia virus (GaLV), monkey immunodeficiency virus (SIV), human immunodeficiency virus (HIV), and combination viruses thereof (Buchscher et al. , J. Virol., 66:2731-2739, 1992; Johann et al., J. Virol., 66:1635-1640 1992; Sommerfelt et al., Virol., 176:58-59, 1990; Wilson et al.., J. Virol., 63:2374-2378, 1989; Miller et al., J. Virol., 65:2220-2224, 1991; PCT/US94/05700).

In the case of transient expression of sucrose phosphorylase, an adenovirus-based system is more widely used. An adenovirus-based vector causes highly efficient transduction in many cells, but does not require cell division. Using the vector, high titers and high levels of expression can be obtained, and can be mass-produced in a simple system. In addition, adeno-associated virus (AAV) vectors are used to transduce cells with target nucleic acids. For example, it is used for the production of nucleic acids and peptides in vitro and for gene therapy in vivo and ex vivo (West et al., Virology., 160:38-47, 1987; U.S. Patent 4,797,368; WO93/24641; Kotin, HumanGene Therapy., 5:793-801, 1994; Muzyczka, J. Clin. Invest., 94:1351, 1994). The construction of a recombinantAAV vector is already known (U.S. Patent No. 5,173,414; Tratschin et al., Mol. Cell. Biol., 5:3251-3260, 1985; Tratschin, et al., Mol. Cell. Biol., 4:20722081, 1984; Hermonat & Muzyczka, PNAS., 81:6466-6470, 1984; Samulski et al., J Virol., 63:038223828, 1989). In clinical trials, a gene transfer method using at least six viral vectors is used, which is an approach to complement the defective vector by inserting a gene into a helper cell line that produces a transducer. pLASN and MFG-S are examples of retroviruses used in clinical trials (Dunbar et al., Blood., 85:3048-305, 1995; Kohn et al., Nat. Med., 1:1017-102, 1995 ; Malech et al., PNAS., 94:(22)12133-12138, 1997). PA317/pLASN was the first therapeutic vector used for gene therapy (Blaese et al., Science., 270:475-480, 1995). The transduction efficiency of the MFG-S packaging vector was 50% or higher (Ellem et al., Immunol Immunother., 44(1):10-20, 1997; Dranoff et al., Hum. Gene Ther. , 1:111-2, 1997).

Recombinant adeno-associated viral vectors (rAAV) are promising alternative gene delivery systems based on defective and non-pathogenic type 2 parvovirus adeno-associated viruses. All vectors are derived from plasmids with AAV 145 bp inverted terminal repeats flanking the transgene expression cassette. Efficient gene delivery and stable transgene delivery due to integration into the genome of the transduced cells are great advantages of this vector system (Wagner et al., Lancet., 351:9117-17023, 1998; Kearns et al., Gene Ther., 9:748-55, 1996).

In another aspect, the present invention relates to a vaccine composition for the treatment or prevention of cancer comprising the polypeptide as an active ingredient.

In the present invention, the vaccine composition induces a Th1 immune response.

In the present invention, the term "Th1 cell" refers to a subset of helper T cell lymphocytes that are characterized in terms of gene expression, protein secretion and functional activity. For example, Th1 cells exhibit a cytokine expression pattern that synthesizes IL-2 and IFN-γ but not IL-4, IL-5, IL-10, and IL-13. Th1 cells are involved in cell-mediated immune responses to various intracellular pathogens, organ-specific autoimmune diseases, and delayed hypersensitivity reactions.

In the present invention, "CTLA-4 (cytotoxic T-lymphocyte-associated protein 4)" is also known as CD152 (Cluster of Differentiation 152) as an immunomodulatory inhibitor, and as a protein receptor acting as an immune checkpoint, it reduces the immune response.

In the present invention, "STING (STimulator of InterferoN Gene, promoter of interferon gene)" refers to a substance exhibiting anticancer effect by activating a signaling pathway related to STING to induce production of inflammatory cytokines including interferon.

In the present invention, the cancer is at least one selected from the group consisting of, for example, squamous cell cancer (e.g., epithelial squamous cell cancer), small cell lung cancer, non-small cell lung cancer, lung cancer, peritoneal cancer, colon cancer, biliary tract tumor, nasopharyngeal cancer, laryngeal cancer, bronchial cancer, oral cancer, osteosarcoma, gallbladder cancer, kidney cancer, leukemia, bladder cancer, melanoma, brain cancer, glioma, brain tumor, skin cancer, pancreatic cancer, breast cancer, liver cancer, bone marrow cancer, esophageal cancer, colon cancer, stomach cancer, cervical cancer, prostate, ovarian cancer, head and neck cancer, and rectal cancer, but is not limited thereto.

The vaccine composition of the present invention is applicable to early cancer.

In the present invention, the term "anti-cancer adjuvant" may be used to increase the anti-cancer effect of the anti-cancer agent, suppress or improve side effects of the anti-cancer agent, and may be administered to a patient in combination with an anti-cancer agent.

In the present invention, the term "prevention" refers to any action of inhibiting or delaying the cancer by administration of a transformant expressing the Hsp 90 epitope of the present invention or a composition comprising the transformant as an active ingredient.

In the present invention, the term "treatment" refers to any action in which a transformant expressing the Hsp 90 epitope of the present invention or a composition comprising the transformant as an active ingredient is administered to stop division or benefit from the cancer or tumor without immortalization.

In the present invention, the composition may be characterized in that it contains the epitopes of heat shock protein 90 represented by the amino acid sequence of SEQ ID NO: 1 and/or 2.

In the present invention, the term "multi-peptide vaccine" is defined to refer to a vaccine containing two or more polypeptide epitopes as described above.

In the present invention, the composition may be characterized in that it further comprises an immune anticancer agent.

In the present invention, the immune anticancer agent is an immune checkpoint inhibitor against any one selected from the group consisting of CTLA-4 (cytotoxic T-lymphocyte-associated protein 4), PD-1 (Programmed cell death protein 1), LAG-3 (Lymphocyte Activation Gene-3), TIM-3 (T- Cell Immunoglobulin and Mucin-domain containing-3), TIGIT (T-cell Immunoreptor with IG and ITIM domain), and VISTA (V-domain Ig Suppressor of T cell Activation), more preferably a CTLA-4 inhibitor, but is not limited thereto.

In the present invention, the composition may further include an anticancer adjuvant, and more preferably, the anticancer adjuvant may be a STING (STimulator of InterferoN Gene) agonist, but is not limited thereto.

In the present invention, the term "immune anticancer agent" is a therapeutic agent that induces immune cells to selectively attack only cancer cells by injecting artificial immune proteins into the body and stimulating the immune system, unlike existing anticancer drugs that attack cancer itself. It is an anticancer drug with a mechanism to restore or strengthen the immune system's ability to recognize or destroy tumors, to overcome the acquired immunosuppression or immune evasion mechanism in cancer cell. The immuno-anticancer agents include, but are not limited to, immune checkpoint inhibitors, immune cell therapy, and immunoviral therapy.

In the present invention, the term "immune checkpoint inhibitor" refer to a kind of immune modulating anticancer drug that supports activating CTL (cytotoxic lymphocytes), when some cancer cells evade bodily immune check point system. Examples thereof include, but are not limited to, a CTLA-4 inhibitor, a PD-1 inhibitor, a LAG-3 inhibitor, a TIM-3 inhibitor, a TIGIT inhibitor, and a VISTA inhibitor.

When using the epitope peptide as a vaccine composition, it is preferable to use the active ingredient in a form mixed with a pharmaceutically acceptable carrier rather than used alone. Here, the pharmaceutically acceptable carrier includes carriers, excipients, and diluents commonly used in the pharmaceutical field.

Pharmaceutically acceptable carriers that can be used in the vaccine composition of the present invention are, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oils.

The vaccine composition of the present invention can be formulated and used in the form of oral dosage forms such as powders, granules, tablets, capsules, suspensions, emulsions, syrups, aerosols, etc., external preparations, suppositories, or sterile injectable solutions according to a conventional method. In the case of formulation, it may be prepared using diluents or excipients such as fillers, extenders, binders, wetting agents, disintegrants, and surfactants that are commonly used. Solid preparations for oral administration include tablets, pills, powders, granules, capsules, etc., and such solid preparations include active ingredients and at least one excipient, such as starch, calcium carbonate, sucrose, lactose, gelatin, etc. In addition to simple excipients, lubricants such as magnesium stearate and talc may also be used. Liquid preparations for oral use include suspensions, liquid solutions, emulsions, syrups, etc. In addition to commonly used diluents such as water and liquid paraffin, various excipients such as wetting agents, sweetening agents, fragrances, and preservatives may be included. Formulations for parenteral administration include sterile aqueous solutions, non-aqueous solutions, suspensions, emulsions, lyophilized formulations, and suppositories. Propylene glycol, polyethylene glycol, vegetable oils such as olive oil, injectable esters such as ethyloleate, and the like may be used as the non-aqueous solvent and suspension. As a base for suppositories, witepsol, tween 61, cacao butter, laurin paper, glycerogelatin, and the like may be used. In particular, in the case of a liquid formulation, it is preferable to sterilize by filtration through a bacteria trapping filter or the like or incorporation of a sterilizing agent. The sterilized composition can be solidified by, for example, lyophilization, and when used, it is dissolved in sterile water or a sterile diluent.

The vaccine composition according to the present invention can be administered to mammals such as cattle, rats, mice, livestock, dogs, humans, etc. by various routes. All modes of administration can be expected, for example, by oral, intravenous, intramuscular, subcutaneous, intraperitoneal injection.

The dosage of the vaccine composition according to the present invention is selected in consideration of the animal's age, weight, sex, and physical condition. The amount required to induce an immunoprotective response in an animal without significant side effects may vary depending on the epitope used as an immunogen and any presence of excipients. In general, at each dose, the immunogenous amount of the polypeptide of the present invention contains 0.1 to 1000 µg of protein, preferably 0.1 to 100 µg, per ml of sterile solution. In the case of a vaccine composition, if necessary, the initial dose can be followed by optionally repeated antigen stimulation.

In another aspect, the present invention relates to a method for treating or preventing cancer, comprising administering the vaccine composition to a cancer patient.

In the present invention, the vaccine composition may be characterized in that it is administered in combination with an immuno-anticancer agent, and it may be characterized in that it is administered in combination with an antibody therapeutic agent.

The immuno-anticancer agent or antibody therapeutic agent used in combination with the vaccine composition of the present invention may be administered simultaneously or sequentially with a time difference and may be selected according to an appropriate time and cycle.

In the present invention, the vaccine composition may be administered in combination with an anticancer agent. The immunological anticancer agents is an immune checkpoint inhibitor against any one selected from the group consisting of CTLA-4 (cytotoxic T-lymphocyte-associated protein 4), PD-1 (Programmed cell death protein 1), LAG-3 (Lymphocyte Activation Gene-3), TIM-3 (T-cell Immunoglobulin and Mucin-domain containing-3), TIGIT (T-cell Immunoreceptor with IG and ITIM domain), and VISTA (V-domain Ig Suppressor of T cell Activation), more preferably CTLA-4 inhibitors, but are not limited thereto.

In the present invention, the vaccine composition may additionally be administered in combination with an anticancer adjuvant, and the anticancer adjuvant may be a STING (STimulator of InterferoN Gene) agonist, but is not limited thereto.

In the present invention, the cancer is at least one selected from the group consisting of for example, squamous cell cancer (e.g., epithelial squamous cell cancer), small cell lung cancer, non-small cell lung cancer, lung cancer, peritoneal cancer, colon cancer, biliary tract tumor, nasopharyngeal cancer, laryngeal cancer, bronchial cancer, oral cancer, osteosarcoma, gallbladder cancer, kidney cancer, leukemia, bladder cancer, melanoma, brain cancer, glioma, brain tumor, skin cancer, pancreatic cancer, breast cancer, liver cancer, bone marrow cancer, esophageal cancer, colon cancer, stomach cancer, cervical cancer, prostate cancer, ovarian cancer, head and neck cancer, and rectal cancer, but is not limited thereto.

In addition, the present invention provides an anticancer composition comprising an epitope of heat shock protein 90 represented by the amino acid sequence of SEQ ID NO: 1 and/or 2.

In addition to the epitope, the composition may further contain other ingredients for stabilizing the active ingredient.

In the present invention, the term "injection" or "administration" may vary depending on the age, sex, weight, etc. of the subject to be administered, and the dose of the vaccine may vary depending on the route of administration, the degree of disease, sex, weight, age, etc. have.

In the present invention, concanavalin A (positive control) activates T cells, but does not activate B cells (when insolubilized, B cells are also activated). In addition, it is used as a means to study the specificity of the membrane structure of cancer cells because many cancer cells exhibit higher aggregation to Con A than normal cells.

In another aspect, the present invention relates to an antibody that specifically recognizes the epitope of heat shock protein 90 represented by the amino acid sequence of SEQ ID NO: 1 or 2.

In the present invention, the antibody may be a monoclonal or polyclonal antibody.

In the present invention, the term "antibody" is a substance produced by stimulation of an antigen in the immune system, also referred to as an immunoglobulin, and specifically binds to a specific antigen to float around the lymph and blood, causing an antigen-antibody reaction. While antibodies exhibit specificity for a specific antigen, immunoglobulins include both antibodies and antibody-like substances that lack antigen specificity. The latter polypeptide, for example, is produced at low levels in the lymphatic system and at increased levels by myeloma. In the present invention, it may be an antibody against an antigen prepared from a gene sequence comprising a sequence encoding a part of the Hsp 90 epitope protein, preferably an antibody against an antigen containing the amino acid sequence of SEQ ID NO: 1, the amino acid sequence of SEQ ID NO: 2, or the amino acid sequence of SEQ ID NO: 1 and SEQ ID NO: 2 in a fused form.

The term "therapeutically effective amount" used in combination with an active ingredient in the present invention refers to an amount effective for preventing or treating a target disease. The therapeutically effective amount of the composition of the present invention may vary depending on various factors, for example, the method of administration, the target site, and the condition of the patient. Therefore, when used in the human body, the dosage should be determined as an appropriate amount in consideration of safety and efficiency. It is also possible to estimate the amount used in humans from the effective amount determined through animal experiments.

Hereinafter, the present invention will be described in more detail through examples. These examples are for illustrative purposes only, and it will be apparent to those of ordinary skill in the art that the scope of the present invention is not construed as being limited by these examples.

### Example 1: Prediction of HSP90-specific MHC class II binding epitope, also known as antigenic determinant using in silico algorithms

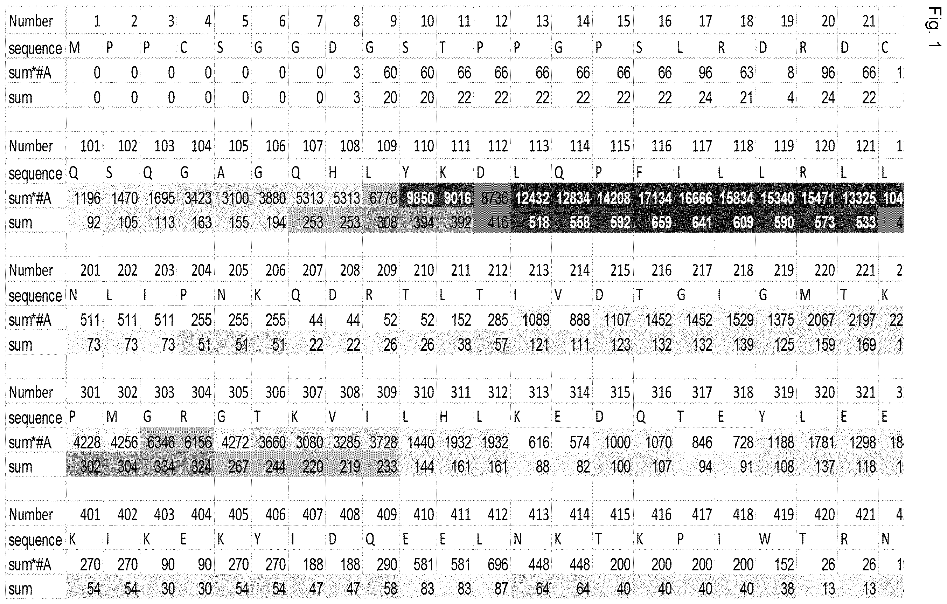

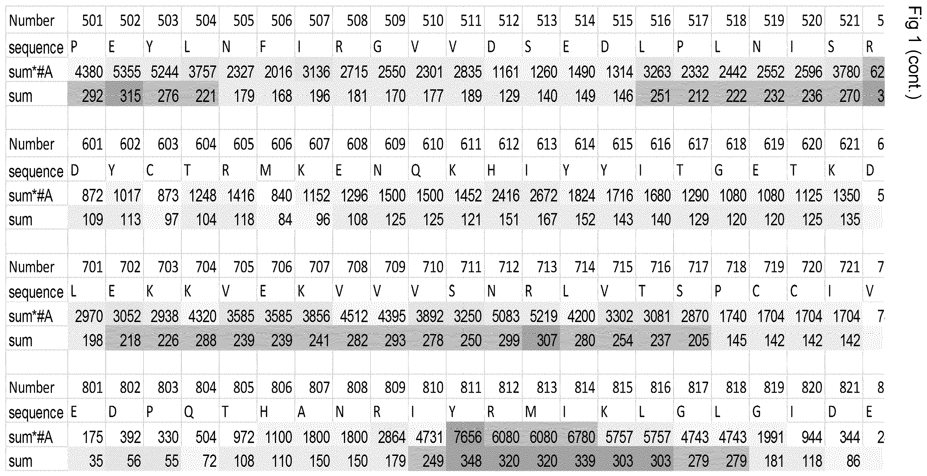

The following 5 computer algorithms were used, in order to select 15-mer peptide sequences that are expected to have good binding affinity to the MHC class II alleles, which are most common in humans, from Heat shock protein 90 (Hsp 90) : SYFPEITHI (Institute for Cell Biology), SYFPEITHI(lnstitute for Cell Biology, Heidelberg, Germany), Propred(lnstitute of Microbial Technology, Chandigarh, India), MHC-Thread(University of Aberdeen, AFPberdeen, United Kingdom), Average Binding matrix method, Rankpep(Harvard, Boston, MA, USA) (Fig. 1). The 15 most common MHC class II alleles are as follows: DRB1*0101, DRB1*1501, DRB1*0301, DRB1*0401, DRB1*0404, DRB1*0405, DRB1*0701, DRB1*0802, DRB1*0901, DRB1*1101, DRB1*1201, DRB1*1302, DRB3*0101, DRB4*0101, DRB5*0101.

Top 12 HSP90-specific peptide sequences were selected in Table 1 below, based on the rank order of the predicted binding affinity. The 12 peptide sequences obtained were synthesized as 15-mer peptides by requesting a peptide synthesis company.

Thereafter, two (p485, p527) peptides represented by the amino acid sequences of SEQ ID NOs: 1 and 2 were selected for multi-peptide vaccine.

Table 1 below shows the selected Hsp 90 15-mer peptide sequences.

**[Table 1]**

| | |
|---|---|
| HSP90 15-mer peptide | amino acid sequences |
| p485-499 | LYVRRVFIMDNCEEL(SEQ ID NO. 1) |
| p527-541 | QSKILKVIRKNLVKK(SEQ ID NO. 2) |
| p109-123 | LYKDLQPFILLRLLM(SEQ ID NO. 5) |
| p145-159 | QAEIAQLMSLIINTF(SEQ ID NO. 6) |
| p160-174 | YSNKEIFLRELISNS(SEQ ID NO. 7) |
| p220-234 | MTKADLINNLGTIAK(SEQ ID NO. 8) |
| p255-269 | QFGVGFYSAYLVAEK(SEQ ID NO. 9) |
| p296-310 | TDTGEPMGRGTKVIL(SEQ ID NO. 10) |
| p328-342 | IVKKHSQFIGYPITL(SEQ ID NO. 11) |
| p457-471 | LEFRALLFVPRRAPF(SEQ ID NO. 12) |
| p582-596 | SELLRYYTSASGDEM(SEQ ID NO. 13) |
| p780-794 | SVKDLVILLYETALL(SEQ ID NO. 14) |

### Example 2: Hsp90-specific immunogenicity of Hsp90 multi-peptide vaccine in an mouse model

To evaluate the immunogenicity of the Hsp 90 multi-peptide vaccine in murine model, six to eight-week-old female FVB mice were divided into 5 mice per group and were immunized subcutaneous (s.c) injection with each Phosphate Buffered Saline (PBS) as a mixture in complete Freund's adjuvant (CFA) / incomplete Freund's adjuvant (IFA) or Hsp 90 multi-peptide vaccine. Three immunizations were given 10 days apart. Ten days after the third vaccination, spleen was extracted from each mouse, to separate splenocytes. Hsp 90 multi-peptide vaccine-specific T cell responses was evaluated by IFN-γ Enzyme-Linked Immunosorbent Spot (ELISPOT) assay.

When preparing and freezing murine splenocytes, the device and materials used are shown in Table 2 below.

**[Table 2]**

| | | | | |
|---|---|---|---|---|
| 15ml and 50ml Falcon tubes, Sterile | Varies | | -- | RT |
| 500ml glass bottles, Sterile | In-house | | -- | RT |
| Bottle top filters, 0.22 micron | Millipore | SCGPS05RE | -- | RT |
| Cell Strainer | BD | 352350 | -- | RT |
| Serological pipettes, Sterile | Varies | | -- | RT |
| Pipetmen and sterile pipetman tips | Varies | | -- | RT |
| 3ml Transfer pipet | BD | 357575 | -- | RT |
| 18G 1.5" hypodermic needle | BD | | -- | RT |
| Sterile square petri dishes | Varies | | -- | RT |
| Centrifuge | Varies | | -- | RT |
| Mr. Frosty containers w/isopropanol | Nalgene | | -- | RT to -80° C |
| -80C Freezer | Varies | | -- | -80° C |
| Water Bath | Varies | | -- | 37° C |
| Liquid Nitrogen storage tank | Varies | | -- | |
| Mouse T Cell Media | In-house | | * | 4° C |
| Freezing Media | In-house | | ** | 4° C |
| ACK Lysis Buffer | In-house | | *** | RT |

To prepare Mouse T Cell Media, 500 mL RPMI-1640 (L-Glut + Hepes) [Mediatech Cellgro], 5 mL penicillin-streptomycin solution [Mediatech Cellgro, #30-002-CI], 50 mL heat-inactivated fetal bovine serum [SAFC Biosciences, if identified, others may be used], 0.5 mL 1000X 2-mercaptoethanol [GIBCO, #21985] were used, and filtering was performed with plasticware. Freezing Media contains 45ml heat-inactivated FBS and 5ml DMSO, and was stored at 4°C after filtering. ACK Lysis Buffer (RBC solution) contains 1L ddH₂O, 8.29g NH₄Cl (final 150mM) and 1g KHCO₃ (final concentration 10mM), is at pH 7.2-7.4, and filtered.

A sterile technique was used for each step of the assay. All experiments were conducted in a tissue culture hood, and if more than 20 splenocytes had to be prepared, steps 9-12 were skipped.

The method of separating splenocytes from the extracted spleen is to take an appropriate amount of Mouse T cell medium in a 50 ml tube, heat it in a 37°C water bath, and then leave only 3 ml medium and fresh spleen in a p100 dish. After that, the spleen was cut into pieces using a blade. the chopped spleen and medium were put in a 50ml tube equipped with a cell strainer. After grinding the spleen on the cell strainer with the end of a 1.5ml tube and dispensing the resultant on the cell strainer with 10ml warm T cell media, the cells were filtered. In addition, cells of a p100 dish were also obtained. Thereafter, centrifugation was performed for 8 minutes at 1,200 rpm. After carefully removing the supernatant, 10ml warm Mouse T cell media is added to resuspend the pellet. Then, after centrifuging for 8 minutes at 1,200 rpm, the supernatant was carefully removed. After suspending with 5 ml of ACK Lysis Buffer, incubation was performed for 5 minutes. Thereafter, 10 ml of Mouse T cell media was added, centrifuged for 8 minutes at 1,200 rpm, and the supernatant was carefully removed as much as possible. Then, 10ml of warm mouse T cell media was added, and 15ul was transferred to a new tube, followed by cell counting. Centrifugation was performed for 8 minutes at 1,200 rpm, and the supernatant was carefully removed.

When using splenocytes on the same day, a medium was added to the cells at a desired concentration, followed by suspension. When the splenocytes were frozen, 2 ml of freeze media was added and resuspended, and then 1 ml each was aliquoted to two tubes. After that, it was stored overnight in a -80 °C freezer and transferred to LN2 the next day.

For IFN-γ Enzyme-Linked Immunosorbent Spot (ELISPOT) assay, the device and materials used are shown in Table 3 below.

**[Table 3]**

| | | | | |
|---|---|---|---|---|
| 15ml and 50ml Falcon tubes,Sterile | Varies | | -- | |
| 500ml glass bottles, Sterile | In-house | | -- | |
| Bottle top filters, 0.22 micron | Millipore | SCGPS05RE | -- | |
| Plastic Wrap | Varies | | -- | |
| Serological pipettes, Sterile | Varies | | -- | |
| Pipetmen and sterile pipetman tips | Varies | | -- | |
| AID Plate Reader and software | AID | | -- | |
| Plate washer | Varies | | -- | |
| 37°C + 5% CO₂ incubator | Nuaire | | -- | |
| Multichannelaspirator (ethanol sterilized) | Varies | | -- | |
| Mouse T cell Media | In-house | | * | 4°C |
| 96-well MAIPS plate, Sterile | MilliPore | MAIPS4510 | | |
| IX PBS, 0.22 *µ*m filtered | In-house | | -- | |
| 35% EtOH (v/v) in deionized water, filter sterilized | In-house | | -- | |
| IX PBS + 0.05% Tween-20, 0.22 *µ*m filtered | In-house | | -- | |
| Streptavidin-HRP | MabTech | 3310-9 | | |
| AEC Staining Kit -OR- AEC Substrate Kit | Sigma OR BD Pharmingen | AEC101-KT OR 551015 | | -20°C |
| Anti-mouse IFNy coating antibody, 1mg/ml | MabTech | AN18 | | 4°C |
| Anti-mouse IFNy biotinylated antibody, 1mg/ml | MabTech | R46A2 | | 4°C |
| Antigens (TT peptide, HSP90 peptide, Concanavalin A) | Varies | | -- | 4°C |
| Conclavin A | Sigma | | C5275 | -80°C |

Mouse T Cell Media was used the same as above.

On Day 0, 30ul of 35% ethanol was incubated for 1 minute in a Prewet MAIPS 96-well plate, and then 200ul of 1xPBS was dispensed and washed 3 times (Ultra purewater and 35% ethanol were purchased from Merck; PBS was used by purchasing 1xPBS). Anti-mouse IFNγ antibody (1mg/ml, stored at 4°C, green cap) was diluted to 10ug/ml using 1xPBS (10ul/ml). The prepared solution was dispensed into each well by 50ul. An amount of 5 ml is required per 96-well plate. The plate lid was closed, covered with a wrap, and incubated for 16 to 24 hours at 4°C (at this time, the wrap must be covered).

After taking out the plate on Day 1, the antibody was removed using a multichannel pipette. Each 200ul of 1xPBS was dispensed and washed three times. In the last wash, PBS was completely removed without damaging the membrane in the plate. 200ul of Mouse T cell media was dispensed into each well and blocked. After incubation for 2 hours in a 37 °C incubator, the plate was labeled on the lid and inside.

Sample Plate Layout:
well A1-A6 (No Antigen)=100uL cells + 100uL Mouse T-cell Media
well A7-A12 (Nonspecific peptide)=100uL cells + 100uL 2X HIV p17
well B1-B6 (Positive Control)=100uL cells + 100uL 2X Conclavin A
well B7-B12 (Vaccine Peptide)=100uL cells + 100uL 2X Peptide

(Continuing for each mouse or pool of cells)

After the concentration of the peptide was doubled in mouse T cell media, 100ul per well could be dispensed and made into n+1 (Table 4)

**[Table 4]**

| antigen | Stock concentration | 2X Working concentration | Final concentration |
|---|---|---|---|
| no ag | - | | - |
| Conclavin A | 1 mg/ml | 5 *µ*g/ml | 2.5 *µ*g/ml |
| peptides | 10 mg/ml | 20 *µ*g/ml | 10 *µ*g/ml |
| peptides | 5 mg/ml | 20 *µ*g/ml | 10 *µ*g/ml |

Thereafter, the blocking media was removed, and 100 ul of the prepared antigen samples were dispensed into appropriate wells each other. Frozen or fresh splenocytes were used for the assay. The method of isolating splenocytes was performed by referring to the SOP IV-101 (Mouse Splenocyte Preparation and Freezing protocol). Splenocytes were resuspended in Mouse T cell media at a final concentration of 3.0x10⁶∼3.5x10⁶ per ml. Using a multi-channel pipette, the counted cells were dispensed at 100 ul per well (3.0x10⁵∼3.5x10⁵). However, 100ul of mouse T-cell media were dispensed for no antigen wells. The plate was incubated for 70-72 hours in a 37 °C incubator. It was confirmed that the color of the medium changed to dark yellow when the cells reacted (Day 1 end).

On Day 4, after incubation for 70-72 hours, the plate was removed from the incubator at 37°C, and the medium of the plate was carefully removed with a multichannel pipette. It was washed twice with 200ul of 1x PBS, and washed three times with 200ul of 1x PBS+0.05% tween-20. However, at the last wash, 1x PBS+0.05% tween-20 was completely removed carefully.

Biotinylated anti-mouse IFNγ antibody (1mg/ml, stored at 4°C, yellow cap) was diluted to 5ug/ml using 1x PBS+0.05% tween-20 (5ul /ml). The prepared solution was dispensed into each well by 50ul. An amount of 5 ml is required per 96-well plate. The plate lid was closed, covered with a wrap, and incubated for 16 to 24 hours at 4°C (must be covered with a wrap) (Day 4 end).

On Day 5, the culture medium from the plate was carefully removed with a multichannel pipette. 200ul of 1x PBS was dispensed and washed 4 times. At the last wash, PBS was completely removed without damaging the membrane in the plate. Streptavidin-HRP was diluted 1:250 in 1x PBS (4ul/ml). The prepared solution was dispensed 50ul into each well. An amount of 5 ml is required per 96-well plate. The plate lid was closed, covered in a wrap, and incubated for 60 minutes at room temperature (the ABC kit was taken out to room temperature during the HRP reaction).

After incubation, the medium from the plate was carefully removed with a multichannel pipette. 200ul of 1x PBS was dispensed and washed 4 times. At the last wash, PBS was completely removed without damaging the membrane in the plate. The lower part of the plate was separated and the water of PBS was removed with a paper towel.

50ul of ABC kit development solution (a small drop mix in 1 ml of a large bottle) was dispensed into each well. An amount of 5 ml is required per 96-well plate. The color of the positive control wells was checked. Development time should not exceed 45 minutes. Usually, the spot is visible between 5-10 minutes, but if the spot is not visible, it is extended to 20-30 minutes. To stop the reaction, it was carefully washed under cold running tap water. It was naturally dried at room temperature in a dark place (at this time, the stop is light-sensitive so it is not exposed to light).

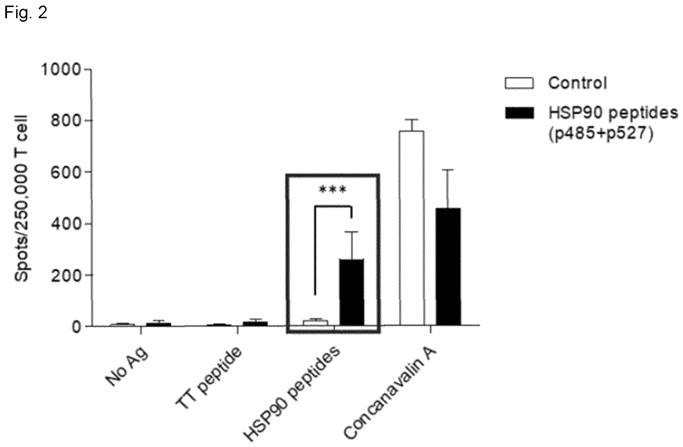

As a result, it was confirmed that in the HSP90 peptide reaction of the FVB mouse model, the Hsp 90 multi-peptide vaccine group exhibited higher specific reactivity by secretion of interferon gamma (IFN-γ) compared to the control group (Fig. 2).

### Example 3: Confirmation of antitumor effect of HSP90 multi-peptide vaccine in mouse model

In order to confirm the anti-tumor effect, a mouse breast cancer cell line (mouse mamary cancer cell-FVB/N-Tg(MMTVneu)-202Mul mouse-derived HER-2 overexpressing breast cancer cell line) was used. MMTVneu transgenic mice 6 weeks old were divided into physiological saline/immune adjuvant group and HSP90 peptide/ immuno adjuvant group into 10 mice per group, and administered 3 times every 10 days, and 10 days after the last administration, the mouse breast cancer cell line was injected subcutaneously into the side of the mouse. The tumor growth was observed every 3 to 4 days, and the tumor size was measured to confirm the antitumor effect of the HSP90 multi-peptide vaccine. The experimental method is the same as in Example 2.

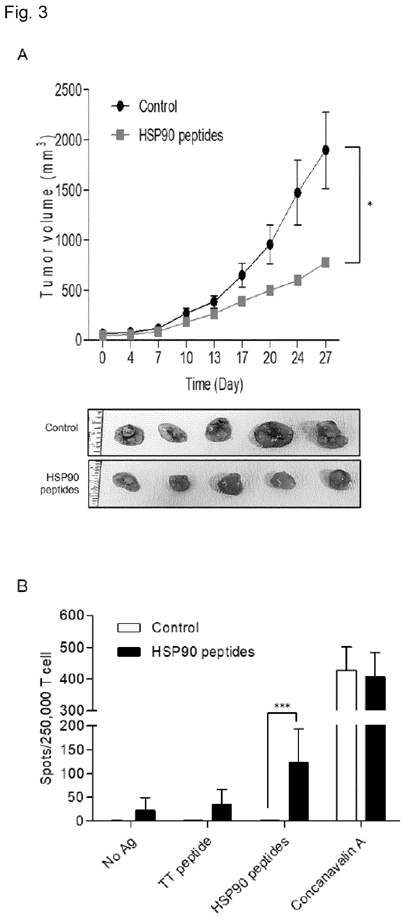

As a result of the experiment, it was confirmed that the tumor size of the Hsp90 multi-peptide vaccine group was significantly reduced compared to the control group in the mouse model (Fig. 3A). In addition, in the HSP90 peptide reaction, it was confirmed that the HSP90 multi-peptide vaccine group exhibited higher specific reactivity by secretion of interferon gamma (IFN-γ) compared to the control group (Fig. 3B).

Therefore, it can be seen that the Hsp90 multi-peptide vaccine composition of the present invention exhibits immunogenicity and has excellent anti-tumor effects.

### Example 4: Confirmation of antitumor effect of combined administration of HSP90 multi-peptide vaccine / STING agonist / CTLA-4 blocker) in a mouse model

### 4.1 Tumor formation and anti-tumor measurements

5 X 10⁵ mouse-derived breast cancer cells were injected subcutaneously into the flank of a 6-week-old female MMTVneu transgenic mouse. After 10 days, 1 control group (PBS+immune adjuvant: Complete Freund's Adjuvant / Incomplete Freund's Adjuvant), and as 4 experimental groups, group 1 HSP90 multi-peptide vaccine group (HSP90 vaccine + immune adjuvant), group 2 (HSP90 vaccine + CTLA- 4 inhibitors; HSP90 vaccine+CTLA-4 inhibitor+immune adjuvant), group 3 (STING Agonist+CTLA-4 inhibitor; STING agonist+CTLA-4 inhibitor+immune adjuvant), group 4 (HSP90 vaccine+STING agonist+CTLA-4) Inhibitors) were injected every 10 days. HSP90 vaccine and STING agonist (DMXAA, 5,6-dimethylxanthenone-4-acetic acid, manufactured by Invivogen) were injected subcutaneously three times and intraperitoneally. Anti-mouse CTLA-4 (CD152, manufactured by Bioxcell) was intraperitoneally injected twice a week until the end of the experiment. During the experiment, tumor growth was measured at 3-4 days intervals. Ten days after the last administration, the spleen of the mouse was excised to isolate splenocytes, and the HSP90 multi peptide-specific T cell response was confirmed using the interferon gamma (IFNγ)-immobilized enzyme antibody method (EezymeLinked Immuno-SPOT assay, ELISPOT). Mouse spleen cells were prepared, frozen, and ELISPOT experimental methods were carried out in the same manner as in Example 2.

### 4.2 Determination of biological markers of HSP90 multi-peptide vaccine effectiveness

As a biological marker of the HSP90 multi-peptide vaccine effect, changes in blood HER2 ECD (Human Epidermal Growth Factor Receptor type 2 ExtraCellular Domain) were analyzed by an enzyme immunoassay (EezymeLinked Immunosorbent assay, ELISA). The specific analysis method is as follows.

### A. Experiment material

a. Carbonate buffer: Add 0.8g Na₂CO₃ and 1.47g NAHCO₃ to a 1 liter beaker, add 400 ml of primary distilled water and dissolve, adjust pH to 9.6, and fill up to 500 ml of primary distilled water. After filtering, it was stored at 4°C.

b. Diluent buffer (1X PBS / 1% BSA): 10g BSA was added to 100ml in 10x PBS, and when dissolved, first distilled water was added to a volume of 1 liter. Stored at 4°C after filtering.

c. Blocking Buffer (1X PBS / 5% BSA): 50g BSA was added to 100ml in 10xPBS, and when dissolved, first distilled water was added to a volume of 1 liter. Stored at 4°C after filtering.

d. Wash Buffer (1xPBS / 0.1% Tween-20): 100ml 10xPBS, 1ml Tween-20, and 899ml of primary distilled water were mixed to prepare a wash buffer.

e. Secondary antibody: Goat anti-mouse IgG (H+L) HRP secondary antibody was purchased from ThermoFisher scientific (cat# 62-6520, volume 1ml) and stored at 4° C., and diluted to 1:10,000 using washing buffer when used.

f. Mouse IgG standard antibody: Mouse IgG standard antibody was purchased from Sigma (cat# I-4506). After purchase, the antibody was made to a final concentration of 2 mg/ml using 150 mM NaCl as a dilution buffer, and stored at -20°C.

g. TMB solution: TMB solution was purchased from Sigma (cat# T0440) and stored at 4°C.

h. 1N HCl stop solution: 18.2 ml of 38% HCI was carefully added little by little to 481.8 ml of primary distilled water to prepare a stock solution and stored at room temperature.

### B. Experimental method and results

ELISA experiments were carried out through the following steps. Steps 1 and 2 were carried out in ice conditions.

2.5 ml of carbonate buffer was dispensed into tube A and 1 ml of carbonate buffer was dispensed into the remaining tubes. Then, 10ul of mouse IgG stock solution (2mg/ml) was added to tube A. Vortexing was performed, and 1 ml was transferred from tube A to tube B (1:1 dilution). Subsequently, dilution was performed 1:1 to L in the order of B→C, C→D (step 1).

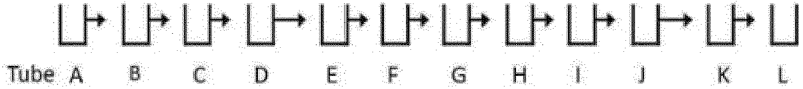
Tube A B C D E F G H I J K L

HSP90 recombinant protein at a concentration of 1 ug/ml was prepared using fresh carbonate buffer. The HSP90 recombinant protein was dispensed 50ul per well into Coat columns #1 to #10. Only carbonate buffer was dispensed into column #11, and the plates were covered with a microseal, followed by antigen-antibody reaction at 4°C overnight (step 2).

Washing was performed three times using a 200 ul washing buffer, and during the third washing, the plate was carefully shaken with a paper towel to completely remove the washing buffer (step 3). Then, 100ul of the blocking solution was dispensed per well and reacted at room temperature for 1 to 2 hours (step 4). The same washing as in step 3 was performed (step 5). Diluting buffer: serum was mixed in a 1:1 ratio in a new 1.5 ml tube, vortexed, and then 50ul was dispensed into each well (step 6). 50ul of dilution buffer was dispensed to plate columns #11 and #12 (step 7). After covering the plate with a microseal, it was reacted at 4° C overnight (step 8). The same washing as in step 3 was performed (step 9). The HRP conjugate antibody was diluted to 1:10,000 using a dilution buffer, and 50ul per well was dispensed, and then reacted with stirring at room temperature for 45 minutes (step 10). The same washing as in step 3 was performed (step 11). After the TMB solution was taken out to room temperature and prepared, 100 ul of the TMB solution was dispensed per well and reacted for 20 minutes at room temperature without light (step 12). 50ul of the reaction stop solution was dispensed per well, respectively (step 13). The fluorescence intensity was measured at 450 nm using a microplate reader device (step 14).

As a result of measuring the change in blood concentration of HER2 ECD after administration of the HSP90 multi-peptide vaccine by an enzyme immunoassay (EezymeLinked Immunosorbent assay, ELISA), the result of using the HSP90 vaccine according to the present invention was low in HER2 ECD

### 4.3 Effect of the combined administration of HSP90 multi-peptide vaccine

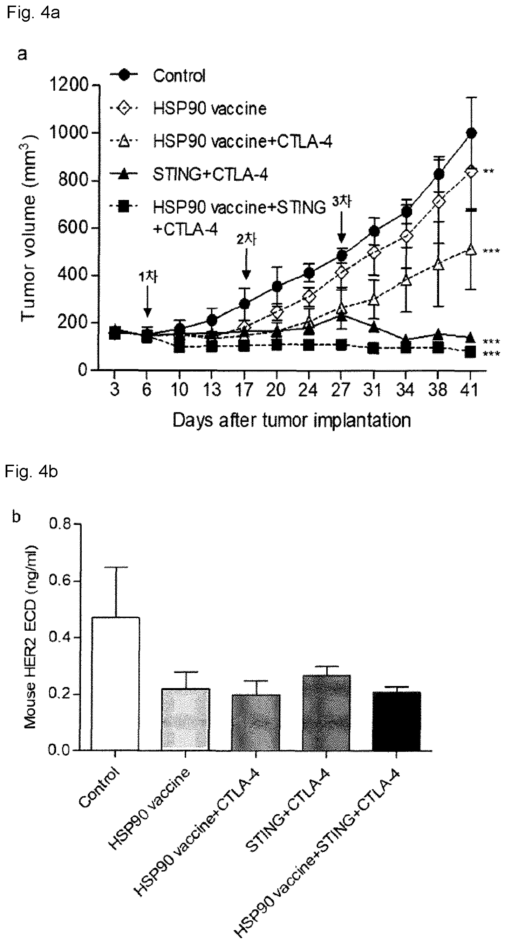

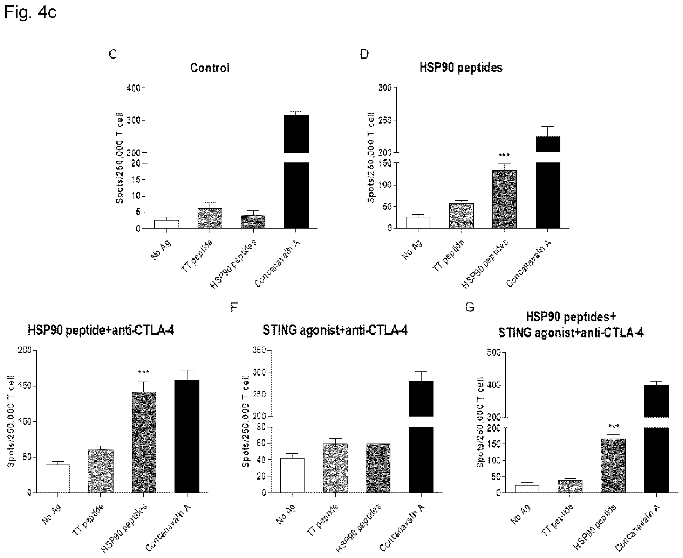

As a result of the experiment, compared to the control group, group 1 (HSP90 multi-peptide vaccine group), group 2 (HPS90 multi-peptide vaccine + CTLA-4 inhibitor), and group 3 (STING agonist + CTLA-4 inhibitor) in the mouse model, group 4 (HPS90 multi-peptide vaccine + STING agonist + CTLA-4 inhibitor) was confirmed that the tumor size significantly decreased when the combination treatment (Fig. 4A). In addition, it was confirmed that the HER2-ECD reactivity decreased in all experimental groups compared to the control group (Fig. 4B). HSP90 peptide addition showed more specific reactivity by secretion of interferon gamma (IFN-γ) in group 4 (HSP90 multiple peptide vaccine + STING agonist + CTLA-4 inhibitor) (Fig. 4C ∼ G). Through this, it can be seen that the HSP90 peptides can be used as a vaccine because the effect of the HSP90 peptide vaccine of the present invention is increased or decreased by combining treatment with STING agonist / CTLA-4 explant.

As described above, specific parts of the present invention have been described in detail, and it will be apparent to those of ordinary skill in the art that these specific techniques are only preferred embodiments, and the scope of the present invention is not limited thereby. Therefore, it will be said that the practical scope of the present invention is defined by the appended claims and their equivalents.

## Claims

1. A polypeptide that is an epitope of heat shock protein 90 represented by the amino acid sequence of SEQ ID NO: 1 or 2.

2. A gene encoding the polypeptide of claim 1.

3. The gene according to claim 2, **characterized in that** it is represented by the nucleotide sequence of SEQ ID NO: 3 or 4.

4. A recombinant vector comprising the gene of claim 2.

5. A recombinant microorganism into which the gene of claim 2 or the recombinant vector of claim 4 has been introduced.

6. A method for preparing the polypeptide of claim 1 comprising the following steps:
(a) culturing the recombinant microorganism of claim 5 to produce the polypeptide of claim 1; and
(b) obtaining the resulting polypeptide.

7. A vaccine composition for the treatment or prevention of cancer comprising the polypeptide of claim 1 as an active ingredient.

8. The vaccine composition of claim 7, wherein the composition comprises all epitopes of heat shock protein 90 represented by the amino acid sequence of SEQ ID NOs: 1 and 2.

9. The vaccine composition of claim 7, wherein the composition further comprises an adjuvant.

10. The vaccine composition of claim 7, wherein the composition further comprises an Immune checkpoint inhibitor against any one selected from the group consisting of CTLA-4 (cytotoxic T-lymphocyte-associated protein 4), PD-1 (Programmed cell death protein 1), LAG-3 (Lymphocyte Activation Gene-3), TIM-3 (T-cell Immunoglobulin and Mucin-domain containing-3), TIGIT (T-cell Immunoreptor with IG and ITIM domain), and VISTA (V-domain Ig Suppressor of T cell Activation).

11. The vaccine composition of claim 9, wherein the composition further comprises an anticancer adjuvant.

12. The vaccine composition according to claim 11, wherein the anticancer adjuvant is a STING (STimulator of InterferoN Gene) agonist.

13. An anticancer composition comprising the polypeptide of claim 1 as an active ingredient.

14. An antibody that specifically recognizes the epitope of heat shock protein 90 represented by the amino acid sequence of SEQ ID NO: 1 or 2.

15. The antibody of claim 14, wherein the antibody is a monoclonal or polyclonal antibody.

16. A method for treating or preventing cancer, comprising administering the vaccine composition of claim 7 to a cancer patient.

17. The method of claim 16, wherein the vaccine composition is administered in combination with an immune anticancer agent.

18. The method of claim 16, wherein the vaccine composition is additionally administered in combination with an anticancer adjuvant.
